# EUROPEAN PATENT APPLICATION

(11) **EP 1 358 884 A1**
(43) Date of publication of application: **05.11.2003**
(21) Application number: 02729381.0
(22) Date of filing: 10.01.2002
(51) Int. Cl.: A61K 31/765, A61P 31/04, A61P 1/00, A23L 1/30

(54) **PREVENTIVES FOR MICROBIAL INFECTIONS**

(30) Priority: 12.01.2001 JP 2001004822
(71) Applicant: AMATO PHARMACEUTICAL PRODUCTS, LTD., Fukuchiyama-shi, Kyoto 620-0932 (JP)
(72) Inventor: MURAKAMI, Masahiro, Osaka-shi, Osaka 547-0026 (JP); TAZUME, Seiki, Isehara-shi, Kanagawa 259-1143 (JP)
(74) Representative: Sternagel, Fleischer, Godemeyer & Partner Patentanwälte
(86) International application number: JP0200078
(87) International publication number: WO02055090

(57) **Abstract**

The object of the present invention is to provide a novel agent for protection against microorganism infection which can be used for protection against infection of pathogenic Escherichia coli such asO-157, or Helicobacter pylori. According to the present invention, there is provided an agent for protection against microorganism infection which comprises a mixture of cyclic and/or straight chain poly lactic acids having a condensation degree of 3 to 20.

## Description

### TECHNICAL FIELD

The present invention relates to an agent for protection against microorganism infection. More specifically, the invention relates to an agent for protection against microorganism infection, which can be used as a medicament, food for special healty uses and a health food that are effective for protection against infection of microorganisms such as pathogenic Escherichia coli such as E. coli O-157 or Helicobacter pylori.

### BACKGROUND ART

Generally, infection of a pathogenic bacterium to a human is established by recognition and binding to a sugar chain structure (receptor) of a complex saccharide present on a surface of a target cell, i. e., an epithelium of the living body. Examples of general therapeutic treatments for infection of pathogenic bacteria to the living body include use of antibiotics. Antibiotics are used for therapy of a disease by extinguishing propagated pathogenic bacteriam, and act at the final stage of infection process of pathogenic bacteria. Therapy of an infectious disease by antibiotics is very effective as a therapy against the living body after onset of the disease. However, due to the nature of antibiotics, there are many problems such as causing various adverse effects and allergic symptoms.

Moreover, various types of antibiotic agents are used for therapy of many bacterial infectious diseases. However, appearance of drug resistant bacteria, particularly MRSA (methicillin-resistant Staphylococcus aureus), caused by frequent uses of these antibiotic agents, is a serious problem in clinical field. In addition, the drug resistant bacteria as well as other opportunistic infection bacterium is a big problem as a nosocomial infectious bacterium.

For therapy of bacterial infectious diseases including opportunistic infection, an antibacterial agent which has been properly selected from many antibacterial agents is used. However, use of the antibacterial agent causes many adverse effects such as shock symptom, renal disorder, hepatic disorder, leukocytopenia, nervous disorder, superinfection, and the like.

As described above, it is still being required to develop an agent for protection against microorganism infection which has a strong protecting action against microorganism infection and shows less adverse effect.

From studies that have been made so far, it has been reported that a mixture of cyclic and/or straight chain poly L-lactic acids having a condensation degree of 3 to 20 is useful as an antineoplastic agent (Japanese Patent Application Laying-Open (Kokai) Nos. 9-227388 and 10-130153). However, evaluation of protecting effect against microorganism infection of a mixture of cyclic and/or straight chain poly L-lactic acids having a condensation degree of 3 to 20, has not been reported.

### DISCLOSURE OF THE INVENTION

An object of the present invention is to provide a novel agent for protection against microorganism infection which can be used for protection against infection of microorganisms such as pathogenic Escherichia coli such as E. coli O-157, and Helicobacter pylori. Further, another object of the present invention is to provide food and drink for protection against microorganism infection using the aforementioned agent for protection against microorganism infection.

In order to study to solve the aforementioned objects, the present inventors have administered a mixture of cyclic and/or straight chain poly lactic acids having a condensation degree of 3 to 20 to axenic mice, and examined a protection effect against infection of E. coli O-157, specifically, a survival ratio of O-157-infected mice and an effect of inhibiting the growth of O-157 in the aforementioned mice. As a result, it has been found that the mixture of poly lactic acids improves the survival ratio of the O-157-infected mice and shows an effect of inhibiting the growth of O-157.

Further, the present inventors have infected Mongolian gerbils with Helicobacter pylori (H.pylori) and fed them for 1 month. Then, the numbers of viable cells in stomach and duodenum of Mongolian gerbil were measured to examine the protection effect of the mixture of cyclic and/or straight chain poly lactic acids having a condensation degree of 3 to 20 against infection of H. pylori. As a result, it has been found that the mixture of poly lactic acids shows a protection effect against infection of H. pylori. The invention was completed on the basis of these findings.

Thus, according to the present invention, there is provided an agent for protection against microorganism infection which comprises a mixture of cyclic and/or straight chain poly lactic acids having a condensation degree of 3 to 20.

The agent for protection against microorganism infection according to the present invention is preferably an agent for protection against bacteria infection, and more preferably it is used as an agent for protection against pathogenic Escherichia coli infection (for example, an agent for protection against pathogenic Escherichia coli O-157 infection) or as an agent for protection against Helicobacter pylori infection. The agent for protection against microorganism infection of the present invention can be used, for example, as a therapeutic agent or a preventive agent of microorganism infection diseases.

Preferably, the lactic acid that is a repeating unit in the poly lactic acid consists substantially of L-lactic acid.

Preferably, the mixture of cyclic and/or straight chain poly lactic acids having a condensation degree of 3 to 20 is a fraction obtained by condensing lactic acids by dehydration under an inactive atmosphere, subjecting the ethanol- and methanol-soluble fractions of the obtained reaction solution to reverse phase column chromatography, and eluting with 25 to 50 weight % acetonitrile aqueous solution of pH 2 to 3 and then with 90 weight % or more acetonitrile aqueous solution of pH 2 to 3.

Preferably, condensation by dehydration is performed by stepwise decompression and temperature rise under nitrogen gas atmosphere.

Preferably, reverse phase column chromatography is performed by ODS column chromatography.

According to another aspect of the present invention, there are provided food and drink for protection against microorganism infection which comprises the aforementioned agent for protection against microorganism infection according to the present invention.

According to still another aspect of the present invention, there is provided the use of a mixture of cyclic and/or straight chain poly lactic acids having a condensation degree of 3 to 20 in the production of an agent for protection against microorganism infection or food and drink for protection from microorganism infection.

According to a still further aspect of the present invention, there is provided a method for giving protection against microorganism infection, which comprises a step of administering an effective amount of a mixture of cyclic and/or straight chain poly lactic acids having a condensation degree of 3 to 20 to mammals such as humans.

### BRIEF DESCRIPTION OF THE DRAWINGS

Fig. shows a mass spectrum of the mixture of poly lactic acids obtained by Production Example 1.
Fig. 2 is a graph showing an effect of CPL on the survival ratio of an O-157-infected mouse.
Fig. 3 is a graph showing an effect of CPL on the number of viable cells in a stool of the O-157-infected mouse.
Fig. 4 is a graph showing a result of measurement of the number of viable cells of H. pylori in a stomach (anterior stomach and posterior stomach) and a duodenum of the CPL-administered group and the control group. An open bar represents the control group (n=5) and a solid bar represents the CPL group (n=6).
Fig. 5 shows an entire view of a positive mode FABMS spectrum of a product obtained in Production Example 2. Range: m/z 10.0000 to 1305.5900
Fig. 6 shows an entire view of a negative mode FABMS spectrum of a product obtained in Production Example 2. Range: m/z 10.0000 to 2000.0000
Fig. 7 shows an enlarged view of a negative mode FABMS spectrum of a product obtained in Production Example 2. Range: m/z 10.0000 to 501.9260
Fig. 8 shows an enlarged view of a mode FABMS spectrum of a product obtained in Production Example 2. Range: m/z 490.2980 to 1003.7700
Fig. 9 shows an enlarged view of a negative mode FABMS spectrum of a product obtained in Production Example 2. Range: m/z 999.9500 to 1504.3400
Fig. 10 shows an enlarged view of a negative mode FABMS spectrum of a product obtained in Production Example 2. Range: m/z 1484.5300 to 2000.0000
Fig. 11 shows an entire view of an NMR spectrum of a product obtained in Production Example 2.
Fig. 12 is a graph showing an effect of CPL on the survival ratio of an O-157-infected mouse.
Fig. 13 is a graph showing an effect of CPL on the number of viable cells in a stool of the O-157-infected mouse.

### THE BEST MODE FOR CARRYING OUT THE INVENTION

The embodiment and method for the practice of the present invention are described in detail below.

The agent for protection against microorganism infection according to the present invention contains a mixture of cyclic and/or straight chain poly lactic acids having a condensation degree of 3 to 20 as an active ingredient, and can be used as an agent for protection against infection of pathogenic Escherichia coli such as E. coli O-157, or Helicobacter pylori. The agent for protection against microorganism infection according to the present invention can be used as a therapeutic agent and a preventive agent for various microorganism infection diseases.

Microorganism in the sense in this specification has its widest sense including all microorganisms such as bacteria and fungi.

Examples of the microorganisms include Escherichia coli (including O-157 and the like), enterococci, staphylococci, Staphylococcus aureus (including methicillin-resistant Staphylococcus aureus), blue pus bacillus (Pseudomonas aeruginosa), Bacterium enterocoliticum (Salmonella enteritidis), Klebsiella pneumoniae, Bacillus subtilis, Candida, Helicobacter pylori and the like. Among these microorganisms, there are species which rot inside intestine, produce a carcinogenic substance or a harmful toxin and also cause various spontaneous infectious diseases.

The agent for protection against microorganism infection according to the present invention has a protecting function against infection of microorganisms such as pathogenic bacteria described above and thus, can be used for the use for prevention or therapy of diseases caused by infection of pathogenic bacteria. Such diseases include diarrhea and food poisoning caused by food-poisoning causal bacteria such as Escherichia coli, caries caused by Streptococcus mutans, and gastric ulcer and gastric cancer caused by Helicobacter pylori.

The agent for protection against microorganism infection according to the present invention can suppress infection of bacteria such as Escherichia coli, Enterococcus faecalis as well as Pseudomonas, pathogenic Escherichia coli and its relative, Salmonella, which cause opportunistic infection, and can suppress growth of bacteria which cause opportunistic infection and enteral infectious pathogenic bacteria. Thus, enteral infection and opportunistic infection can be prevented.

Among these bacteria, E. coli is a bacterial species constituting an enteral flora of a healthy person. Some E. coli population which acquired an ability of producing a special pathogenic factor causes an enteral infectious disease in human. In 1996, epidemic outbreak of the infectious disease caused by enteral bleeding E. coli (EHEC) O-157: H7 occurred in many places in Japan. Since then, case number of epidemic outbreak reduced. However, some cases occurred sporadically. EHEC produces, as a major pathogenic factor, Vero toxin which shows a strong cytotoxicity against a Vero cell derived from a renal cell of an African green monkey, and when infected, causes 5% serious cases with complication of hemolytic uremic syndrome and rarely of cerebropathia. The agent for protection against microorganism infection according to the present invention can be particularly used for prevention and therapy of the infectious diseases caused by pathogenic E. coli O-157.

Also, it has been known that Helicobacter pylori causes gastritis and also works as recurrent and healing-delay factor of gastroduodenal ulcer. In addition, a relationship between this species and gastric cancer has been suggested. The agent for protection against microorganism infection according to the present invention can be used for protection against infection of Helicobacter pylori.

The agent for protection against microorganism infection according to the present invention is effective for protection against infection of pathogenic bacteria and thus, has a preventive action in that a person who previously ingests the agent before infection becomes hard to be infected with bacteria, and if infected, becomes easy to be cured. Therefore, it is preferred to daily ingest the agent for protection against microorganism infection according to the present invention as a health food and a medicament.

In the agent for protection against microorganism infection and the food and drink for protection against microorganism infection according to the present invention, a mixture of cyclic and/or straight chain poly lactic acids having a condensation degree of 3 to 20 is used as an active ingredient.

The term "a mixture of poly lactic acids" used in the present invention means a mixture wherein cyclic and/or straight chain poly lactic acids having a condensation degree of 3 to 20 are present at any ratio. That is to say, the term "mixture" does not only mean a mixture of poly lactic acids having any condensation degree ranging from 3 to 20, but is also used as a concept including a mixture of cyclic and straight chain poly lactic acids. As is described below in the present specification, "a mixture of poly lactic acids" can be obtained by condensing lactic acids by dehydration and then performing purification by a suitable method. Although the term "a mixture of poly lactic acids" is used in the present specification for the sake of convenience, this term also includes a poly lactic acid consisting of a single ingredient such as a cyclic poly lactic acid having single condensation degree or a straight chain poly lactic acid having single condensation degree.

The term "condensation degree" is used to mean the number of lactic acid unit that is a repeating unit in poly lactic acids. For example, the cyclic poly lactic acid is assumed to have the following structural formula wherein n represents condensation degree (n = 3 to 20).

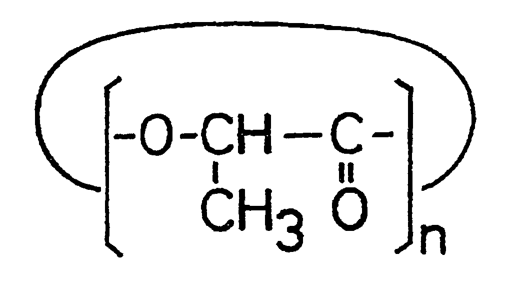

When "lactic acid" is simply referred to in the present specification, this lactic acid includes all of L-lactic acid, D-lactic acid or a mixture comprising these types of lactic acid at any ratio. Preferably in the present invention, the lactic acid consists substantially of L-lactic acid. The term "substantially" is used herein to mean that the ratio of L-lactic acid units in a mixture of poly lactic acids (number of L-lactic acid unit / number of L-lactic acid unit + number of D-lactic acid unit × 100) is, for example, 70% or more, preferably 80% or more, more preferably 85% or more, further more preferably 90% or more, and particularly preferably 95% or more. The ratio of L-lactic acid units in a mixture of poly lactic acids depends on the ratio of L-lactic acid and D-lactic acid that exist in lactic acids used as a starting substance.

The methods for producing a mixture of cyclic and/or straight chain poly lactic acids having a condensation degree of 3 to 20 are not particularly limited, and the mixture of poly lactic acids can be obtained by the production methods described, for example, in Japanese Patent Application Laying-Open (Kokai) Nos. 9-227388 and 10-130153 or Japanese Patent Application No. 11-39894 (All publications cited herein are incorporated herein by reference in their entirety).

More specifically, for example, a mixture of cyclic and/or straight chain poly lactic acids having a condensation degree of 3 to 20 can be obtained by the following method A.

### Method A:

First, lactic acid (preferably, lactic acid substantially consisting of L-lactic acid) is condensed by dehydration under an inactive atmosphere. Examples of the inactive atmosphere include nitrogen gas and argon gas, and nitrogen gas is preferred.

Dehydration and condensation reaction is carried out at a temperature of 110°C to 210°C, preferably 130°C to 190°C under normal pressure to reduced pressure of approximately 1mmHg, and particularly preferably the reaction is carried out by stepwise decompression and stepwise temperature rise. A reaction period can be determined as appropriate. For example, the reaction can be carried out for 1 to 20 hours. Where stepwise decompression and stepwise temperature rise are applied, reaction is performed by dividing the reaction period into two or more partial reaction periods, and then determining pressure and temperature for each of the reaction periods. Where stepwise decompression is applied, pressure can be reduced, for example, from a normal pressure to 150mmHg and then to 3mmHg. Where stepwise temperature rise is applied, temperature can be raised, for example, from 145°C to 155°C and then to 185°C. Practically, the reaction can be carried out by using these conditions in combination, for example, 145°C, normal pressure, 3 hours; 145°C, 150mmHg, 3 hours; 155°C, 3mmHg, 3 hours; and 185°C, 3mmHg, 1.5 hours.

Subsequently, ethanol and methanol are added to the reaction mixture obtained by the dehydration and condensation reaction, and the mixture is filtered. The obtained filtrate is dried to obtain ethanol- and methanol-soluble fractions. The term "ethanol- and methanol-soluble fractions" is used in the present specification to mean fractions soluble in a mixed solution of ethanol and methanol. In order to obtain ethanol and methanol-soluble fractions, a reaction mixture obtained by dehydration and condensation reaction is mixed with ethanol and methanol, where the ratio of ethanol and methanol can be determined as appropriate. For example, the ratio is ethanol:methanol = 1 : 9. The order, method and the like for adding ethanol and methanol to a reaction mixture are not limited, and may be selected as appropriate. For example, ethanol may be added at first to the reaction mixture obtained by the dehydration and condensation reaction, and then methanol may be added thereto.

The thus obtained ethanol- and methanol-soluble fractions are subjected to reverse phase column chromatography, especially to chromatography where an octadecylsilane (ODS) column is used. First, fractions eluted with 25 to 50 weight % acetonitrile aqueous solution of pH 2 to 3 are removed, and then fractions eluted with 90 weight % or more acetonitrile aqueous solution of pH 2 to 3, preferably 99 weight % or more acetonitrile aqueous solution, are collected so as to obtain a mixture of cyclic and/or straight chain poly lactic acids having a condensation degree of 3 to 20.

The thus obtained mixture of cyclic and/or straight chain poly lactic acids is neutralized with an alkaline substance such as sodium hydroxide, and is dried under reduced pressure, and then according to standard techniques, the mixture can be formulated in a desired form as mentioned below.

Other examples of the methods for producing a mixture of cyclic and/or straight chain poly lactic acids having a condensation degree of 3 to 20 used in the present invention include a method described in Japanese Patent Application No. 11-265715 (hereinafter referred to as method B), or a method described in Japanese Patent Application No. 11-265732 (hereinafter referred to as method C) (All publications cited herein are incorporated herein by reference in their entirety). Methods B and C will be described specifically below.

### Method B:

Method B is a method for producing a cyclic lactic acid oligomer which comprises polymerizing lactid in the presence of a lithium compound represented by RYLi [wherein R represents an aliphatic group or aromatic group, Y represents oxygen atom or sulfur atom]. In the case of performing the polymerization reaction, the ratio of the amounts of the lithium compound (RYLi) is 1-0.1 mol, preferably 0.2-0.3 mol per mol of lactide. The reaction temperature is -100 to 0°C, preferably -78 to -50°C. Reaction is preferably carried out by starting from a temperature of -78 to -50°C and gradually raising it to room temperature. The reaction is preferably carried out in the presence of a reaction solvent. As the reaction solvent, there can be used ,for example, a cyclic ether such as tetrahydrofuran, diethylether, and dimethoxyethane. The reaction atmosphere can be an inactive gas atmosphere such as nitrogen gas and argon. The reaction pressure is not limited, and is preferably a normal pressure.

The composition (that is, the mixing ratio of cyclic lactic acid oligomer and a chain lactic acid oligomer) of the lactic acid oligomer obtained as described above fluctuates depending on the lithium compound used as a reaction assistant. Where a lithium compound of alkyl alcohol having a carbon number of 1 to 3 (ROLi) (wherein R represents an alkyl group with carbon number 1 to 3) is used as a lithium compound, a mixture of a cyclic lactic acid oligomer and a chain oligomer (proportion of the cyclic lactic acid oligomer: 80 to 85 weight %) is obtained. When a lithium compound of alkyl alcohol having a carbon number of 4 or more such as t-butyl alcohol, or thiophenol compound is used as a lithium compound, substantially only a cyclic lactic acid oligomer can be selectively obtained.

### Method C:

This method comprises:
(i) a first heating step which comprises heating lactic acid under a pressure condition of 350 to 400 mmHg and to a temperature of 120 to 140°C so as to perform dehydration and condensation, and distilling off and removing only by-product water without distilling lactid off;
(ii) a second heating step for synthesizing a product condensed by dehydration comprising chain lactic acid oligomers as the main ingredient, which comprises, after completion of the first heating step, heating the reaction product to a temperature of 150 to 160°C while reducing the reaction pressure to 15 to 20 mmHg at a decompression rate of 0.5 to 1 mmHg/min, wherein only by-product water is distilled off and removed while avoiding distillation of lactid; and after the reaction pressure is reduced to 15 to 20 mmHg, maintaining the reaction under the same pressure condition and at a reaction temperature of 150 to 160°C;
(iii) a third heating step for synthesizing cyclic oligomers which comprises, after completion of the second heating step, heating under a pressure condition of 0.1 to 3 mmHg and at 150 to 160°C to cyclize the chain lactic oligomer.

In this method, first, in the first heating step, lactic acid is heated under reduced pressure to perform dehydration and compression reaction. In this case the reaction period is 3 to 12 hours, preferably 5 to 6 hours. To allow the reaction in the first heating step to proceed smoothly, by-product water produced by condensation of lactic acids by dehydration is distilled off. At this time, distillation of by-product water is performed such that lactid, which is the dehydrated condensed product of two molecules of lactic acid, is not distilled off. To achieve such purpose, the reaction pressure is maintained at a reduced pressure, preferably 300 to 500 mmHg, more preferably 350 to 400 mmHg. Under this pressure condition, heating is performed at a temperature range of 100 to 140°C, preferably 130 to 140°C. The reaction product produced by reaction in the first heating step mainly comprises as the main ingredient a dehydrated condensed product of 3 to 23 molecules of lactic acid.

To obtain oligomers having an increased average degree of polymerization in the second heating step after completion of the above first heating step, heating is performed at a temperature higher than the reaction temperature of the above first heating step, preferably at 145°C to 180°C, more preferably 150°C to 160°C, while the reaction pressure is reduced to 10 to 50 mmHg, preferably 15 to 20 mmHg, so that dehydration and condensation reaction is further continued.

As with the reaction in the above first heating step, reaction is performed under a condition where by-product water, but not lactid, is distilled off, to allow the reaction to proceed smoothly. The rate at which reaction pressure is reduced to a pressure in the above range (decompression rate) is normally required to be maintained within a range of 0.25 to 5 mmHg/min, preferably 0.5 to 1 mmHg/min, in order to avoid distillation of lactid and increase the reaction efficiency. A decompression rate lower than the above range is not preferred because it will increase the time required to reduce pressure to a given pressure. On the other hand, a decompression rate higher than the above range is also not preferred because it will cause lactid to be distilled off together with by-product water.

After the reaction pressure is reduced to a certain pressure, reaction is further continued at that reaction pressure. The heating time period in this case is 3 to 12 hours, preferably 5 to 6 hours.

A lactic acid oligomer having an average polymerization degree of 3 to 30, preferably 3 to 23 is obtained by the reaction in the above second heating step. The proportion of cyclic oligomers in the oligomers in this case is normally about 70 to 80 weight %.

In the third heating step, after completion of the above second heating step, a reaction pressure is maintained at 0.25 to 5 mmHg, preferably 0.5 to 1 mmHg, and reaction is further continued at a temperature of 145 to 180°C, preferably 150 to 160°C. A reaction period is 3 to 12 hours, preferably 5 to 6 hours. By-product water produced in this case is also distilled off. In this case, distillation of lactid is preferably avoided. However, since the reaction product contains almost no lactid, it is not required to specially lower the decompression rate.

Lactic acid oligomers produced by reaction in the above third heating step have an average polymerization degree of 3 to 30, preferably 3 to 23, and contain cyclic oligomer in the proportion of 90 weight % or more, preferably 99 weight % or more.

The above methods A, B and C merely show some of specific examples of methods of producing a mixture of poly lactic acids used in the present invention. A mixture of poly lactic acids which is produced by other methods can also be used in the present invention.

The agent for protection against microorganism infection according to the present invention can be prepared by optionally selecting and using a component or an additive used in the formulation of medicaments, quasi-drugs, cosmetics and the like as necessary without impairing the effect of the present invention in addition to the aforementioned essential component. The agent for protection against microorganism infection according to the present invention can be used as single medicaments, and also can be contained and used in medicaments, quasi-drugs, cosmetics for skin and hair and the like.

The dosage form of the agent for protection against microorganism infection according to the present invention is not particularly limited, and any form suitable for the purpose can be selected from dosage forms for oral or parenteral administration.

Examples of dosage forms suitable for oral administration include a tablet, a capsule, a powder, a drink, a granule, a parvule, a syrup, a solution, an emulsion, a suspension, a chewable tablet, and the like. Examples of dosage forms suitable for parenteral administration include, but are not limited to, an injection (e.g. a subcutaneous, intramuscular or intravenous injection, and the like), an external preparation, a drop, an inhalant, an air spray, dose drops, eye drops.

Liquid formulations suitable for oral administration such as a solution, emulsion or syrup can be produced using water; sugars such as sucrose, sorbit or fructose; glycols such as polyethylene glycol or propylene glycol; oils such as sesame oil, olive oil or soybean oil; antiseptics such as p-hydroxybenzoate; and flavors such as strawberry flavor and peppermint. In order to produce solid formulations such as capsule, tablet, powder or granule, there can be used an excipient such as lactose, glucose, sucrose or mannite; a disintegrator such as starch or sodium alginate; a lubricant such as magnesium stearate or talc; a binder such as polyvinyl alcohol, hydroxypropylcellulose or gelatin; a surfactant such as fatty acid ester; and a plasticizer such as glycerine, and the like.

Formulations for an injection or drop that is suitable for parenteral administration preferably comprise, as an active ingredient, the above substance in a dissolved or suspended state in a sterilized aqueous medium which is isotonic to the recipient's blood. For example, in the case of an injection, a solution can be prepared using an aqueous medium consisting of a saline solution, a glucose solution or a mixture of a saline solution and a glucose solution. In the case of a formulation for intestinal administration, it can be prepared using carriers such as theobroma oil, hydrogenated lipids or hydrogenated carboxylic acid, and can be provided as a suppository. In order to produce an air spray, the above substance as an active ingredient may be dispersed as microparticles, and a carrier which does not irritate the recipient's cavitas oris and respiratory tract mucosa and which facilitates absorption of the active ingredient can be used. Specific examples of carriers include lactose, glycerine, and the like. Formulations having a form such as aerosol or dry powder may be prepared depending on the properties of the substance of an active ingredient and the carrier to be used. One or two or more auxiliary ingredients selected from glycols, oils, flavors, an antiseptic, an excipient, a disintegrator, a lubricant, a binder, a surfactant, a plasticizer and the like may be added to these formulations for parenteral administration.

The dose and dosage frequency of the agent for protection against microorganism infection according to the present invention are determined as appropriate, depending on various factors such as purpose of administration, dosage form, condition such as age, body weight or sex of a patient. Generally, the dose of an active ingredient per day is 1 to 10,000 mg/kg, preferably 10 to 2000 m/kg, and more preferably 10 to 200 mg/kg. It is preferred that the above dose of the agent is dividedly applied about once to 4 times per day.

The time of administration of the agent for protection against microorganism infection according to the present invention is not particularly limited, and the agent can be administered before or after infection.

The present invention also relates to food and drink for protection against microorganism infection, which comprises a mixture of cyclic and/or straight chain poly lactic acids having a condensation degree of 3 to 20. Thus, the mixture of cyclic and/or straight chain poly lactic acids having a condensation degree of 3 to 20 which is used in the present invention is used not only as a form of single agent as mentioned above, but also may be mixed into food and drink, and used.

The preparation form of the food and drink for protection against microorganism infection according to the present invention is not particularly limited, so long as a mixture of poly lactic acid can be contained without being decomposed.

Specific examples of products of food and drink for protection against microorganism infection according to the present invention include health foods or supplements including drinks, such as those generally called a soft drink, drinkable preparation, health food, specified supplement food, functional food, function-activating food, nutritional supplementary food, supplement, feed, feed additive and the like.

Specific examples of food and drink include confectionary, such as a chewing gum, chocolate, candy, sweet tablet, jelly, cookie, biscuit and yogurt; frozen deserts, such as ice cream and sherbet; beverages, such as tea, soft drink (including juice, coffee, cocoa and the like), nutrition supplement drinkable preparation, and cosmetic drinkable preparation; and all other food and drink, such as bread, ham, soup, jam, spaghetti, and frozen food. Alternatively, the mixture of poly lactic acids used in the present invention can also be used by adding to seasoning, food additives, and the like. A protecting effect against microorganism infection is exerted by ingesting the food and drink for protection against microorganism infection according to the present invention, and there can be provided safe food and drink which show substantially no toxic side effect.

The food and drink for protection against microorganism infection according to the present invention can be obtained by directly mixing and dispersing a mixture of poly lactic acids in a common raw material used in food, and then processing the mixture into a desired form by a know method.

The food and drink for protection against microorganism infection according to the present invention encompasses food and drink in every form, and the types are not specifically limited. That is, the food and drink can be provided by mixing the agent for protection against microorganism infection according to the present invention into the above-mentioned various food and drink, or various nutrient compositions, such as various oral or enteral nutrient preparations or drinks. Compositions of such food and drink may include protein, lipid, carbohydrate, vitamin and/or mineral, in addition to the mixture of cyclic and/or straight chain poly lactic acids having a condensation degree of 3 to 20. The form of the food and drink is not specifically limited, and may be in any form, such as solid, powdery, liquid, gel, and slurry forms, so far as it is in a form that is easily ingested.

The content of the mixture of poly lactic acids in the food and drink is not specifically limited, and is generally 0.1 to 20 weight %, more preferably approximately 0.1 to 10 weight %.

The mixture of poly lactic acids is preferably contained in the food and drink in an amount which achieve a protecting effect against microorganism infection which is an object of the present invention. Preferably, about 0.1 g to 10 g, more preferably about 0.5 g to 3 g, of the mixture of poly lactic acids is contained per food or drink to be ingested.

The content of the specification of Japanese Patent Application No.2001-4822 which the present application claims a priority based on is incorporated herein by reference as a part of the disclosure of the present specification.

The present invention is further described in the following examples, but the scope of the present invention is not limited by the examples in any way.

### EXAMPLES

### Production Example 1: Production of a mixture of poly lactic acids (hereinafter referred to as CPL)

500 ml of L-lactic acid (to which D-lactic acid was also mixed) was placed into a separable flask in a mantle heater. 300ml/min of nitrogen gas was flowed therein while stirring. Accumulated water was introduced into a flask equipped with a reflux condenser via a warmed descending type connecting tube, while heating at 145°C for 3 hours. Furthermore, after pressure was reduced to 150 mmHg and heated at the same temperature for 3 hours, the mixture was heated at 155°C for 3 hours under a reduced pressure of 3 mmHg, and then at 185°C for 1.5 hours under a reduced pressure of 3 mmHg to obtain poly lactic acids as a reaction product.

The obtained poly lactic acids were kept at 100°C, and 100ml of ethanol and 400ml of methanol were separately added thereto, and then the mixture was allowed to be cooled. This mixture was added to 500ml of methanol, and the mixture was well stirred and left to stand. Then, the mixture was filtrated for purification. The filtrate was subjected to vacuum drying and then dissolved in acetonitrile to obtain 200ml (stock solution) in total.

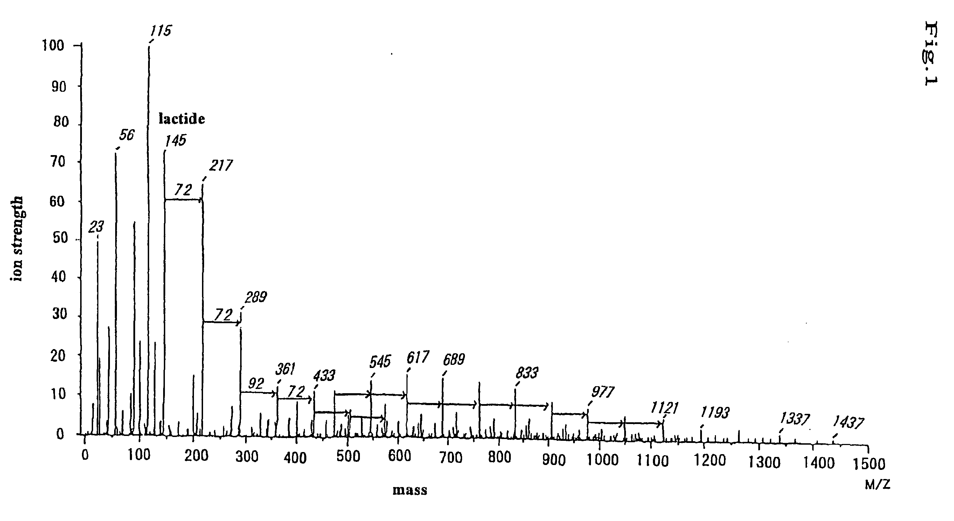

The stock solution was subjected to a reverse phase ODS column (TSK gel ODS-80 TM) which was previously equilibrated, and was stepwise eluted with 30%, 50% and 100% acetonitrile (pH2.0) each containing 0.01M hydrochloric acid to obtain poly lactic acids (condensation degree of 3 to 20) as an acetonitrile 100% elution fraction. The mass spectrum of the obtained substance is shown in Fig. 1. As is clear from the regular fragment ion peaks in Fig. 1, the obtained mixture of poly lactic acids mainly comprises cyclic condensate, and a small amount of linear condensate is contained therein.

### Test example 1:

### (Materials and method)

The experimental animals used were 5 to 12-week old axenic BALB/c line mice (CLEA JAPAN INC). For the CPL group, the standard solid diet (CE2) mixed with 1% CPL (prepared in Production Example 1) was previously autoclaved (121°C for 10 minutes), and was given to the mice ad lib together with sterilized tap water. For the control group, the standard solid diet (CE2) sterilized by radiation (cobalt 60) was given to the mice ad lib together with sterilized tap water. All mice used for the experiment were kept and managed in a vinyl isolator.

EHEC (8.5 x 10⁶ cfu/mouse) was orally administered by using a stomach tube at third day after CPL administration was started. Then, the viability was observed and the number of the bacteria in stool was measured every day.

For the measurement of the number of bacterium, stool was collected and weighted, and 10-folds volume of a phosphate buffer solution (PBS) was added thereto and the mixture was homogenized. Then, stepwise dilution was carried out and 0.1 ml of the stepwise-diluted solution was applied to a normal agar medium (Defco, USA), and the number of the colonies was counted.

### (Result and Discussion)

### (1) Effect of CPL on the viability of EHEC-infected mice

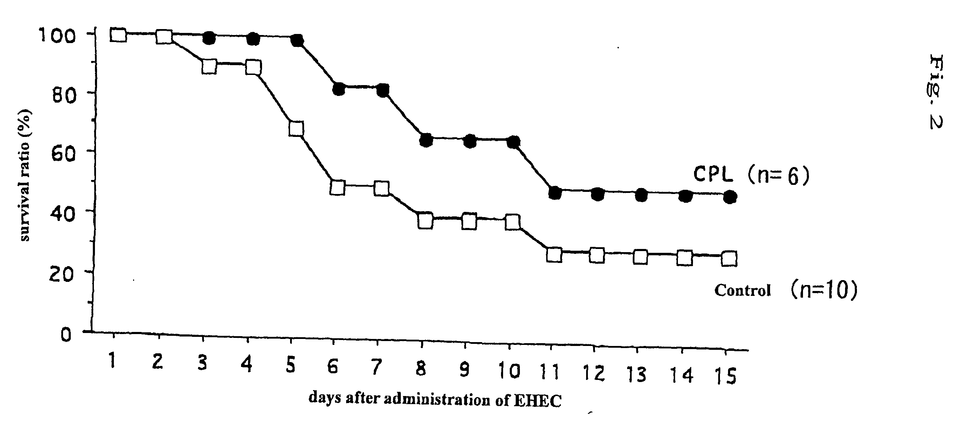

Fig. 2 shows the result of observation of viability (survival ratio) of the CPL administered group and the control group. In the axenic mice which received the standard solid diet mixed with CPL for 3 days before EHEC administration, death was observed stating at the 6th day after administration of EHEC and the survival ratio at the 11th day was 50%. It was found that a resistance of the CPL-administered group against EHEC was higher as compared with the infected mice of the no CPL-administered group where the death was observed from the 3rd day and the survival ratio at 11th day was 30%.

### (2) The number of viable cells in stool

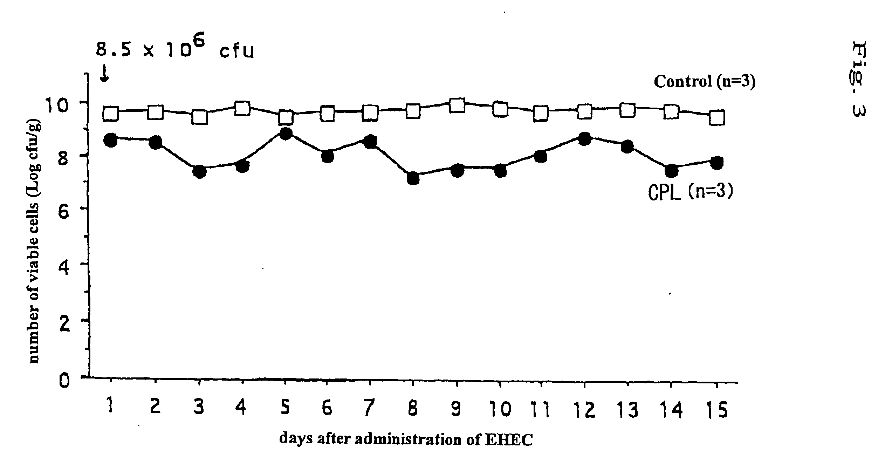

Fig. 3 shows the number of viable cells in a stool of the CPL administered group and the control group. During the period of observation of viability, EHEC which was excreted in stool was continuously detected. It was found that EHEC stayed in an enteron of the axenic mice and that the number of the viable cells per 1g of wet stool of the CPL-administered group was somewhat fewer during the period of measurement as compared with that of the no CPL-administered group.

From the result as described above, it was demonstrated that the death of the infected mice was caused by infection of EHEC and that the mice which took the standard solid diet mixed with CPL showed a resistance against infection of EHEC.

### Test Example 2:

### (Materials and method)

The experimental animals used were 9 to 12-week old Mongolian gerbils (SEYAKKU). For the CPL group, the standard solid diet (CE2) mixed with 0.2% CPL was given to the mice ad lib together with sterilized tap water. For the control group, the standard solid diet (CE2) sterilized by radiation (cobalt 60) was given to the mice ad lib together with sterilized tap water. H. pylori (1.5 to 3.0 x 10⁶ cfu/mouse) was orally administered continuously by using the stomach tube for three day, and CPL contained in the diet was given. At the 30th day, the animals were sacrificed, and the stomach and the duodenum were exenterated, and the number of viable cells was measured. For the measurement of the number of viable cells, the stomach (divided into anterior stomach and posterior stomach) and the duodenum were weighed, 10-folds volume of phosphate buffer solution (PBS) was added, and the mixture was homogenized. Then, stepwise dilution was carried out and 0.1 ml of the stepwise-diluted solution was applied to a Helicobacter agar medium (Nissui Pharmaceutical Co., Ltd), and the number of the colonies was measured.

### (Result and Discussion)

### Number of viable cells in the stomach and the duodenum of Mongolian gerbil infected with H. pylori

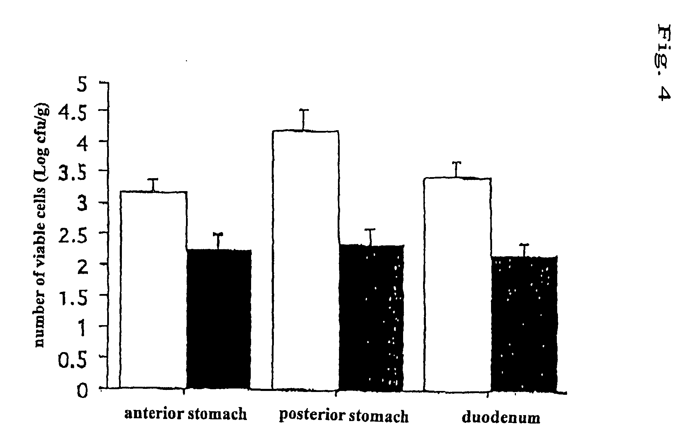

Fig. 4 shows the result of the measurement of the number of viable cells of H. pylori in the stomach (the anterior stomach and the posterior stomach) and the duodenum of the CPL administered group and the control group. The numbers of viable cells of the anterior stomach (10² cfu), the posterior stomach (10² cfu) and the duodenum (10² cfu) of the CPL administered group showed a decreasing tendency in comparison with the numbers of viable cells of the anterior stomach (10³ cfu), the posterior stomach (10⁴ cfu) and the duodenum (10⁴ cfu) of the control group.

From the result as described above, it was found that CPL itself or its metabolic product suppressed the growth of H. pylori in the living body and the administration of CPL could give a protection against infection of H. pylori.

### Production Example 2: Preparation of a mixture of lactic acid oligomer (hereinafter referred to as X03)

A reaction scheme of Production Example 2 is shown below.

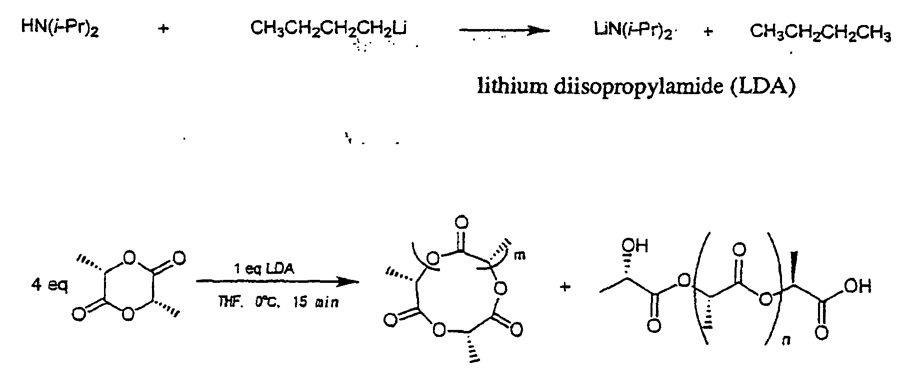

To 5 mL of THF solution containing 0.101 g (1 mmol) of diisopropylamine was added 0.63 mL (1 mmol) of n-butyl lithium (1.6 M hexane solution) at 0°C under nitrogen atmosphere, and the mixture was stirred for 10 minutes to prepare lithium diisopropylamide (LDA). Then, 4 mL THF solution containing 0.577 g (4 mmol) of L-(-)-lactide was added thereto, and the mixture was stirred for 15 minutes to be reacted. To this reaction mixture was added 20 mL of a saturated aqueous solution of ammonium chloride to treat the reaction, and 10 mL of water was further added. Extraction was carried out 5 times with THF (50 mL), and the organic layer was dried with anhydrous sodium sulfate. Anhydrous sodium sulfate was filtered out and then, the organic solvent was subjected to vacuum concentration to afford 0.53 g of a crude product. To the resultant crude product was added 6 mL of ether, and the mixture was dipped in an ultrasonic washer for 10 minutes, and filtered to afford 0.39 g of a white solid product having a melting point of 125 to 129°C.

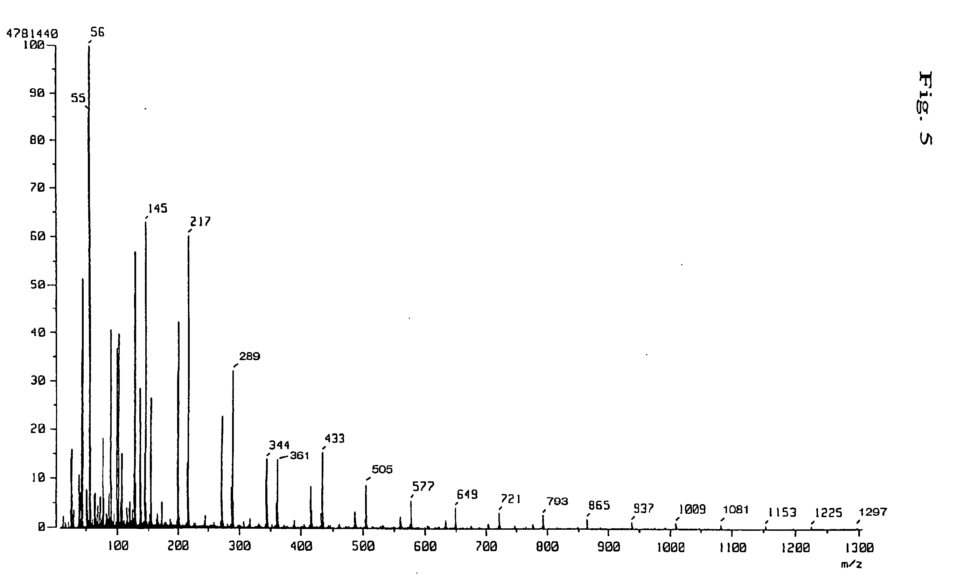

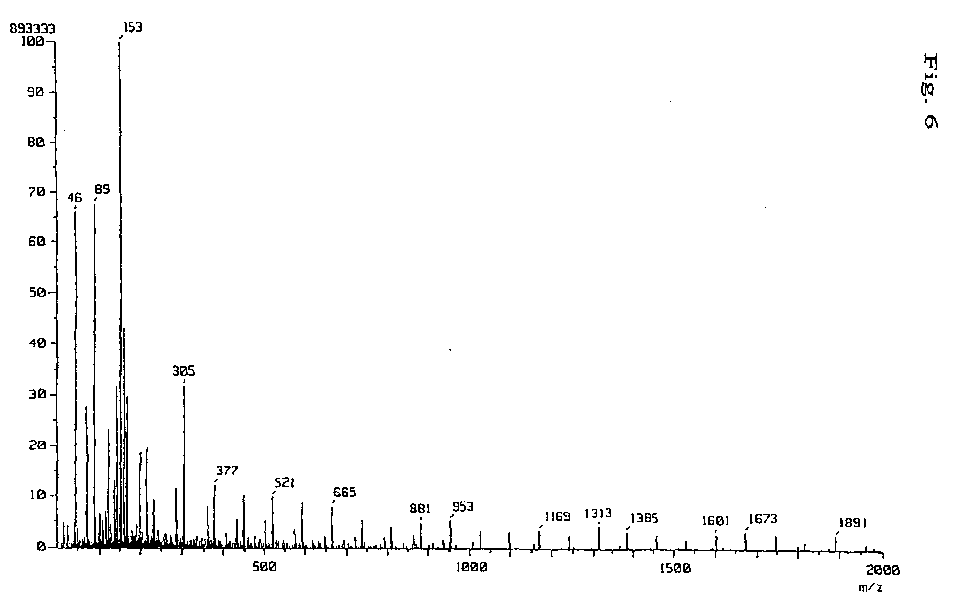

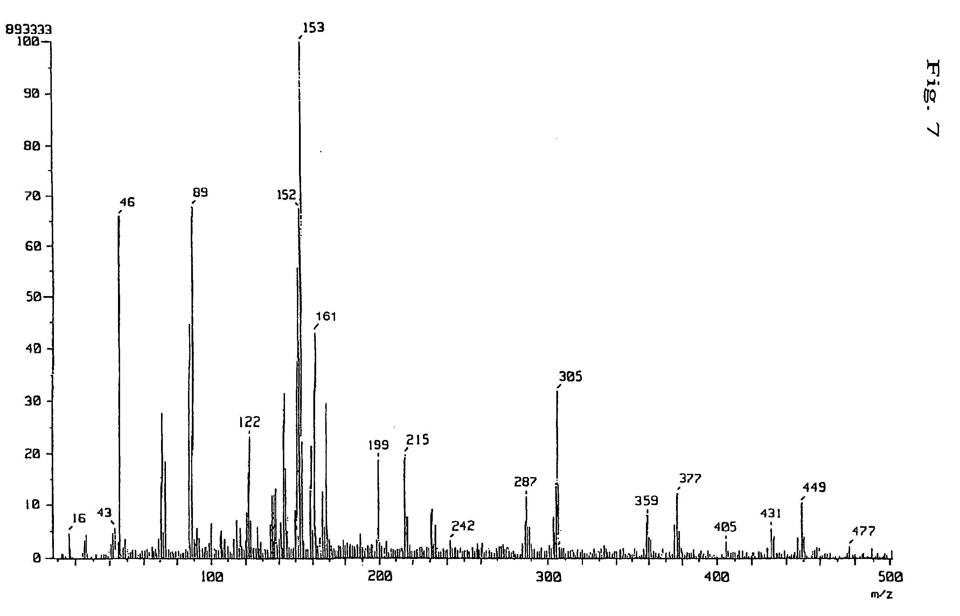

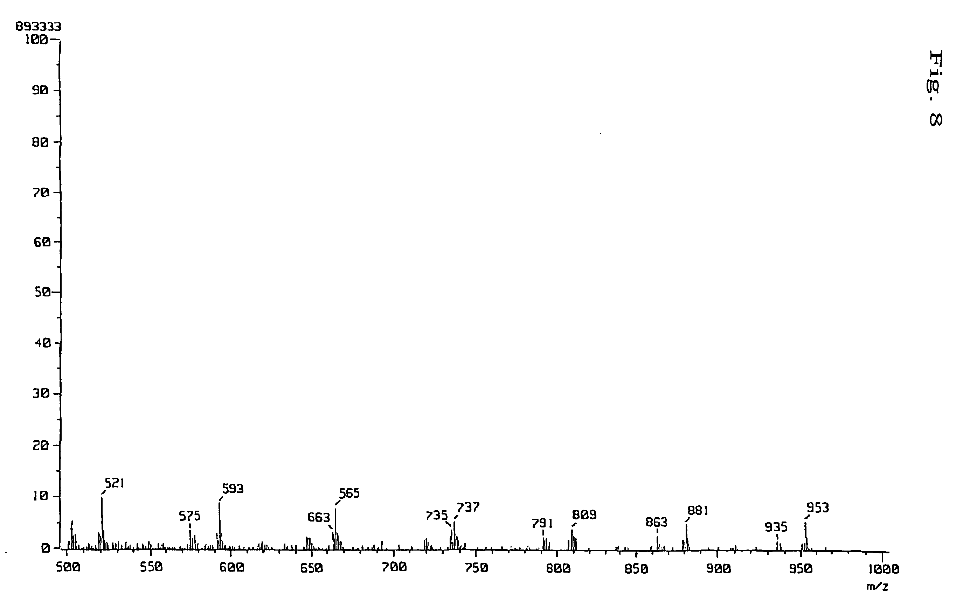

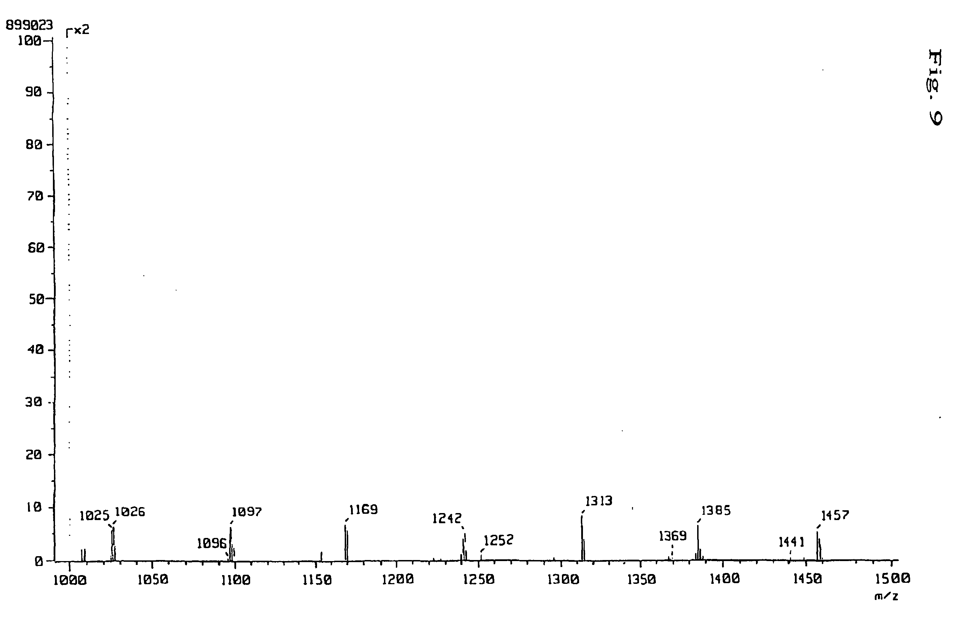

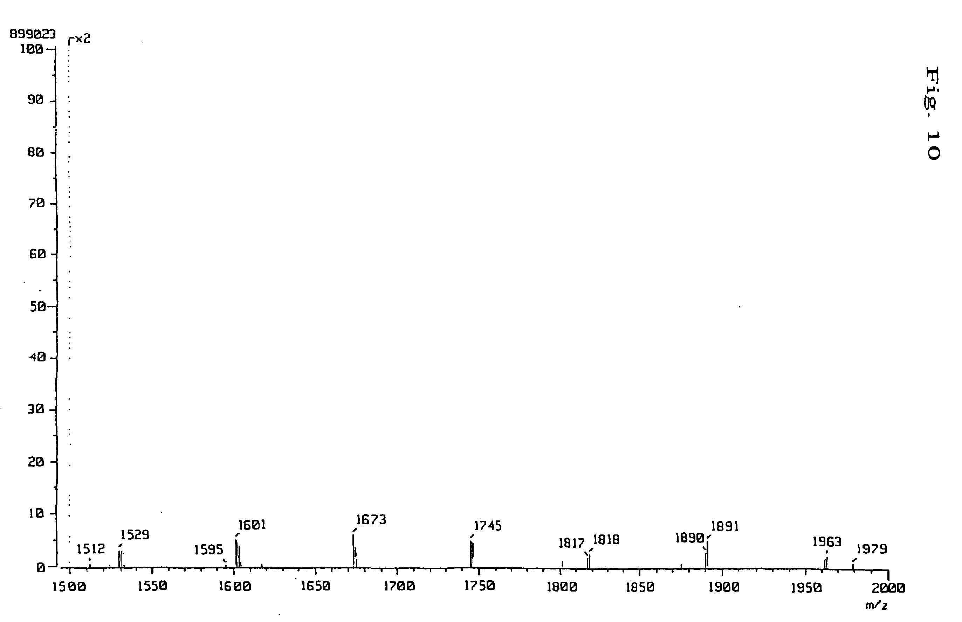

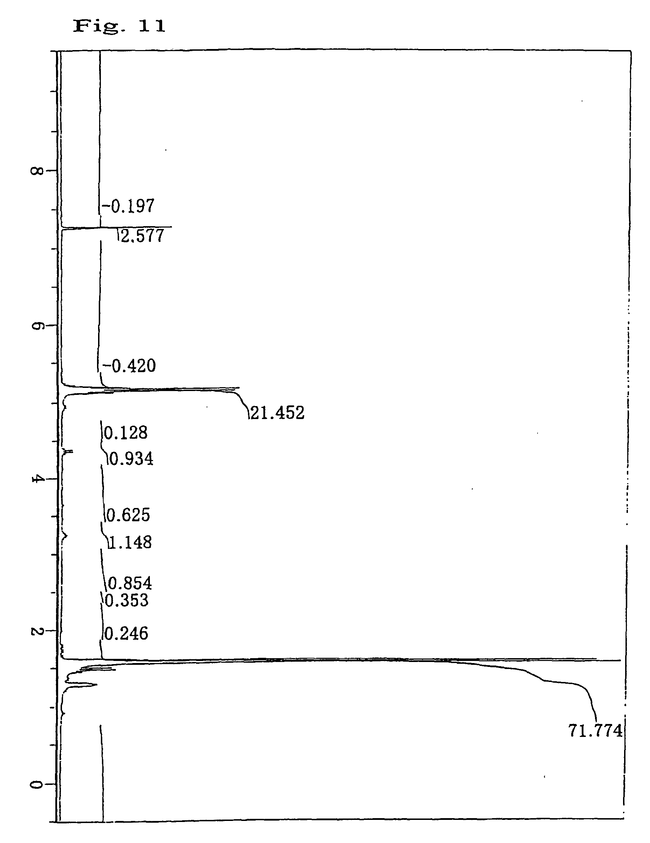

Figs. 5 to 11 show physical data of the obtained product. From the FABMS and NMR data shown in Figs. 5 to 11, it was confirmed that trimer to 21mer of a cyclic lactic acid oligomer and trimer to 27mer of a linear lactic acid oligomer were present in the solid product.

### Test Example 3:

### (Materials and method)

The experimental animals used were 5 to 6-week old axenic BALB/c line mice (CLEA JAPAN INC). For the CPL group, the standard solid diet mixed with 0.1% X03 (prepared in Production Example 2) (CE2 + X03) was previously autoclaved (121°C for 10 minutes), and was given to the mice ad lib together with sterilized tap water. For the control group, the standard solid diet (CE2) sterilized by radiation (cobalt 60) was given to the mice ad lib together with sterilized tap water. All mice used for the experiment were kept and managed in a vinyl isolator.

EHEC (5.0 x 10⁶ cfu/mouse) was orally administered by using a stomach tube at the 7th day after CPL administration was started. Then, the viability was observed and the number of the bacteria in stool was measured every day.

For the measurement of the number of bacterium, stool was collected and weighted, and 10-folds volume of a phosphate buffer solution (PBS) was added thereto and the mixture was homogenized. Then, 0.1 ml of the solution was applied to a normal agar medium (Defco, USA), and the number of the colonies was counted.

### (Result and Discussion)

### (1) Effect of CPL on survival ratio

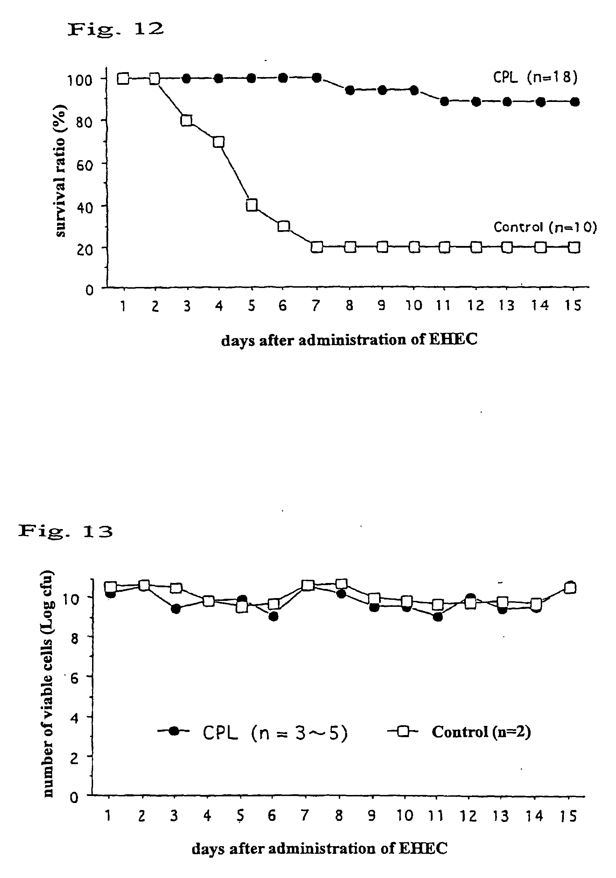

Fig. 12 shows the result of observation of viability (survival ratio) of the CPL administered group and the control group. The survival ratio of EHEC-infected mice during observation for 15 days was 20% (2/10) in the control group and 88.9% (2/18) in the CPL-administered group.

### (2) Number of viable cells in stool

Fig. 13 shows the result of the measurement of the number of viable cells in the stool of the CPL administered group and the control group. The numbers of viable cells per 1g of wet stool were 10⁹⁻¹⁰ cfu in the CPL administered group and the control group.

### Industrial Applicability

The agent for protection against microorganism infection according to the present invention can be used for therapy and prevention of infection diseases of microorganism (for example, pathogenic Escherichia coli such as O-157 and Helicobacter pylori).

The mixture of poly lactic acids used in the invention as an active ingredient is a low condensate of lactic acids derived from organism components, and therefore shows a high biocomparability and few side effects.

## Claims

1. An agent for protection against microorganism infection which comprises a mixture of cyclic and/or straight chain poly lactic acids having a condensation degree of 3 to 20.

2. The agent for protection against microorganism infection according to claim 1 which is an agent for protection against bacteria infection.

3. The agent for protection against microorganism infection according to claim 1 or 2 which is an agent for protection against pathogenic Escherichia coli infection.

4. The agent for protection against microorganism infection according to claim 1 or 2 which is an agent for protection against pathogenic Escherichia coli O-157 infection or an agent for protection against Helicobacter pylori infection.

5. The agent for protection against microorganism infection according to any of claims 1 to 4 which is used as a therapeutic agent or a preventive agent of microorganism infection diseases.

6. The agent for protection against microorganism infection according to any one of claims 1 to 5, wherein the lactic acid that is a repeating unit in the poly lactic acid consists substantially of L-lactic acid.

7. The agent for protection against microorganism infection according to any one of claims 1 to 6, wherein the mixture of cyclic and/or straight chain poly lactic acids having a condensation degree of 3 to 20 is a fraction obtained by condensing lactic acids by dehydration under an inactive atmosphere, subjecting the ethanol- and methanol-soluble fractions of the obtained reaction solution to reverse phase column chromatography, and eluting with 25 to 50 weight % acetonitrile aqueous solution of pH 2 to 3 and then with 90 weight % or more acetonitrile aqueous solution of pH 2 to 3.

8. The agent for protection against microorganism infection according to claim 7, wherein condensation by dehydration is performed by stepwise decompression and temperature rise under nitrogen gas atmosphere.

9. The agent for protection against microorganism infection according to claim 7 or 8, wherein reverse phase column chromatography is performed by ODS column chromatography.

10. Food and drink for protection against microorganism infection, which comprises the agent for protection against microorganism infection according to any of claims 1 to 9.
